# EUROPEAN PATENT APPLICATION

(11) **EP 4 710 860 A1**
(43) Date of publication of application: **18.03.2026**
(21) Application number: 25201907.0
(22) Date of filing: 12.09.2025
(51) Int. Cl.: A61B 6/46, A61B 6/03

(54) **AUTOMATIC WINDOWING USING ORGAN CLASSIFICATION**

(30) Priority: 16.09.2024 US 202418885924
(71) Applicant: Siemens Healthineers AG, 91301 Forchheim (DE)
(72) Inventor: YEREBAKAN, Halid, Carmel, IN, 46033 (US); HERMOSILLO VALADEZ, Gerardo, West Chester, PA, 19382 (US); SOZA, Grzegorz, 90562 Heroldsberg (DE)
(74) Representative: HKW Intellectual Property PartG mbB

(57) **Abstract**

Systems and methods include determining first pixel intensities based on Hounsfield Unit (HU) values of a first set of a plurality of voxels of a volume and a first HU window, presenting pixels having the first pixel intensities on a display, determining a position of a cursor on the display, determining an anatomical structure corresponding to the position of the cursor, determining a second HU window based on the anatomical structure, the second HU window being different from the first HU window, determining second pixel intensities of the pixels based on the HU values of the first set of the plurality of voxels and the second HU window, and changing the first pixel intensities of the displayed pixels to the second pixel intensities.

## Description

### BACKGROUND

Computed Tomography (CT) imaging generates three-dimensional images of anatomical structures within a patient. Generally, an X-ray tube and a detector are rotated around a patient to obtain projection images from various projection angles with respect to the patient. A three-dimensional CT image is reconstructed from the projection images using known techniques.

Each voxel of a CT image is associated with a Hounsfield Unit (HU) value. An HU value of a voxel is obtained from a linear transformation of attenuation coefficients measured by the detector. An HU value represents the radio density of tissue located at a voxel relative to water, which is assigned an HU value of zero.

In order to display a CT image, the HU values of the voxels are converted to pixel intensities. Conventional displays represent pixel intensities using 8-bit values, resulting in 256 possible pixel intensity values. Since the range of radiodensities of human tissue is between -1024 HU and 3071 HU (i.e., 4096 possible values), a mapping function is required to convert HU values to pixel intensity values.

Windowing is commonly used to convert values from one range to another. In one example, an HU window is defined as having a center value of 1500 and a size of 500. Accordingly, the HU window spans 1000 HU to 2000 HU. Based on this HU window, HU values greater than 2000 HU map to a pixel intensity of 255, and HU values less than 1000 HU map to a pixel intensity of 0. The 254 remaining pixel intensity values (i.e., 1 to 254) may be linearly distributed among the 1001 HU values between 1000 HU and 2000 HU.

Due to different radiodensity distributions within different anatomical structures, different HU windows are often preferred for viewing different anatomical structures. For example, an operator may prefer to view lung tissue using a window size of 1600 HU at window center -600 HU in order to delineate features within the lung tissue, and a to view liver tissue using a window size of 160 HU at window center 60 HU. These preferences may differ among operators.

Conventionally, an operator specifies a desired HU window size and window center by manipulating a user interface using a keyboard and/or pointing device. For example, an operator first controls the user interface to navigate to an image slice which includes a region of interest. Next, the operator executes one action to define the HU window size and another action to define the HU window center. The intensities of the display pixels are determined based on the HU values of the voxels being displayed and on the defined HU window size and center. The pixels are then displayed with the determined intensities.

Systems are desired to efficiently and automatically determine and apply suitable HU windows to display different anatomical structures while viewing a CT image.

### BRIEF DESCRIPTION OF THE DRAWINGS

FIG. 1 is a block diagram illustrating determination and application of different HU windows during viewing of a CT image according to some embodiments.
FIGS. 2A through 2C illustrate anatomical structures displayed using different HU windows according to some embodiments.
FIG. 3 is a flow diagram of a process to determine and apply different HU windows during viewing of a CT image according to some embodiments.
FIG. 4 is a block diagram illustrating determination and application of different HU windows based on segmented organ data according to some embodiments.
FIG. 5 is a block diagram illustrating determination and application of different HU windows using an organ classification model according to some embodiments.
FIG. 6 is a tabular representation of organ- and user-specific HU window information according to some embodiments.
FIG. 7 is a block diagram of an imaging system according to some embodiments.

### DETAILED DESCRIPTION

The following description is provided to enable any person in the art to make and use the described embodiments. Various modifications, however, will remain apparent to those in the art.

Generally, some embodiments determine HU windows based on the position of a cursor within a displayed image. An anatomical structure located at the position is determined, and an HU window corresponding to the anatomical structure is determined. The HU window is then used to convert the HU values underlying the displayed image to new pixel intensity values, and an image consisting of the new pixel intensity values is displayed. Embodiments may thereby provide a seamlessly-changing adaptive HU window which is particularly suited for display of an anatomical structure of interest.

Determination of the anatomical structure may comprise submitting intensity values of pixels proximate to the cursor position to a classification model. In other embodiments, determination of the anatomical structure comprises comparing a location of a voxel corresponding to the cursor position to organ locations of a previously-generated organ segmentation map. Moreover, an HU window corresponding to a given anatomical structure may differ for different operators.

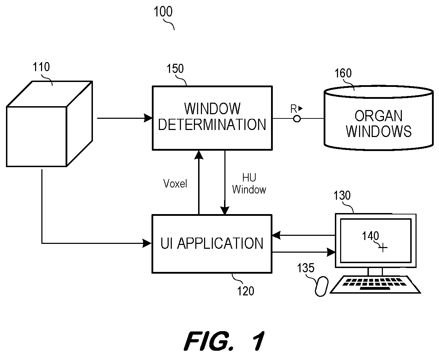

FIG. 1 is a block diagram of system 100 to determine and apply different HU windows during viewing of an image according to some embodiments. The illustrated components of system 100 may be implemented in computer hardware, in program code and/or in one or more computing systems executing such program code as is known in the art. Such a computing system may include one or more processing units which execute program code stored in one or more non-transitory storage media. More than one functional component may be implemented by a single computing system in some embodiments. One or more of the computing systems may comprise a virtual machine, and one-or more computing systems may comprise a cloud-based compute resource providing on-demand scalability and failure recovery.

A single computing system (e.g., a computer workstation) may include each of components 120 through 160 in some embodiments. User interface (UI) application 120 may receive CT image 110 and control display thereof on display 130. For example, an operator may instruct UI application 120 to open CT image 110 and display a slice of CT image 110. In response, UI application 120 converts the HU values of voxels of the slice into pixel intensities suitable for display on display 130. An operator may further instruct UI application 120 to display a different slice, to change the HU window used to convert the HU values to pixel intensity values, and to perform any other known viewing functions.

User interface (UI) application 120 may receive CT image 110 and control display thereof on display 130. For example, an operator may instruct UI application 120 to open CT image 110 and display a slice of CT image 110. In response, UI application 120 converts the HU values of voxels of the slice into pixel intensities suitable for display on display 130. An operator may further instruct UI application 120 to display a different slice, to change the HU window used to convert the HU values to pixel intensity values, and to perform any other known viewing functions.

According to some embodiments, an operator may control pointing device 135 (e.g., a mouse) to move cursor 140 on display 130 while display 130 is presenting a portion of CT image 110. Cursor 140 is overlaid on the presented portion and therefore overlays an anatomical structure depicted within the portion.

UI application 120 tracks the position of cursor 140 as it moves on display 130. UI application 120 determines a voxel of CT image 110 which corresponds to the position (i.e., which is represented by a pixel located at the position) and provides an identifier of the voxel to window determination component 150. Based on the voxel, window determination component 150 determines an anatomical structure (e.g., an organ) on which cursor 140 is currently overlaid.

Window determination component 150 determines an HU window based on the determined anatomical structure. According to some embodiments, component 150 performs a lookup on organ windows data 160 based on an identifier of the anatomical structure. Organ windows data 160 may associate identifiers of various anatomical structures with respective HU windows (e.g., specified as a window center and a window size). Organ windows data 160 may include, for different operators, different respective HU windows for a same anatomical structure. Therefore, the lookup on organ windows data 160 may be based on an identifier of the determined anatomical structure and an identifier of a current operator of pointing device 135.

Window determination component 150 returns the HU window to UI application 120. In response, UI application 120 converts the currently-displayed voxels of CT image 110 to pixel intensities using the returned HU window and displays an image of the pixel intensities on display 130. The process may repeat as described above. That is, UI application may continue to track a position of cursor 140 and, as the position moves with respect to the displayed portion of CT image 110, the voxel corresponding to the position is provided to window determination component 150 to determine a new HU window (or to determine that the HU window should remain unchanged).

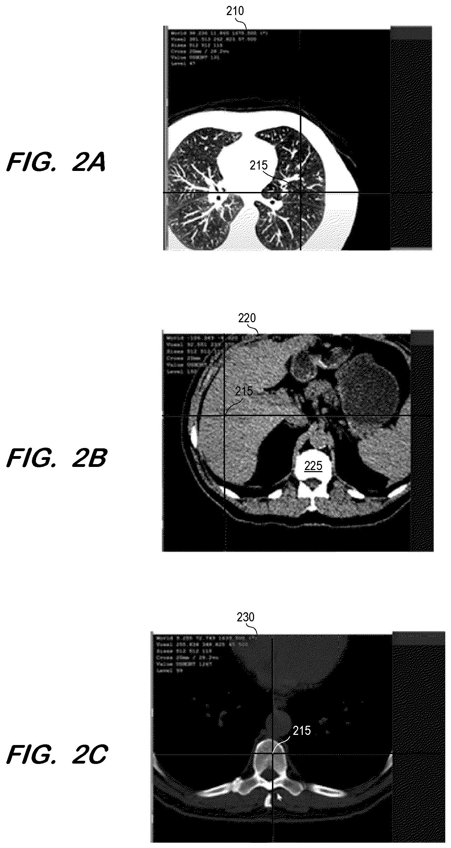

FIGS. 2A through 2C illustrate anatomical structures displayed using different HU windows according to some embodiments. The pixels of each image 210, 220, 230 represent a particular slice of voxels of three-dimensional CT data. The HU values of the voxels have been converted into pixel intensities of their representative pixels.

Referring to image 210 of FIG. 2A, cursor 215 is positioned at a pixel location representing lung tissue. By virtue of the above-described system, the pixel intensities of image 210 are automatically converted from corresponding HU values based on an HU window which provides suitable visibility of lung tissue.

As shown in FIG. 2B, an operator has operated a UI application to display a different slice of CT data. Moreover, cursor 215 has moved to a new position of the display. The pixels at the new position are determined to represent liver tissue. Accordingly, the pixel intensities of image 220 are automatically determined based on an HU window which provides suitable visibility of liver tissue.

It is noted that pixels 225 of image 220 represent bone. Due to the HU window used to determine the pixel values of image 220, pixels 225 exhibit a maximum intensity (e.g., 255). In contrast, FIG. 2C shows cursor 215 at a new position including pixels representing bone. The pixel intensities of image 230 are therefore automatically determined based on an HU window which provides suitable visibility of bone (i.e., having a greater window center value than the HU window used in image 220), resulting in a more detailed view of the composition of the bone than shown in image 220.

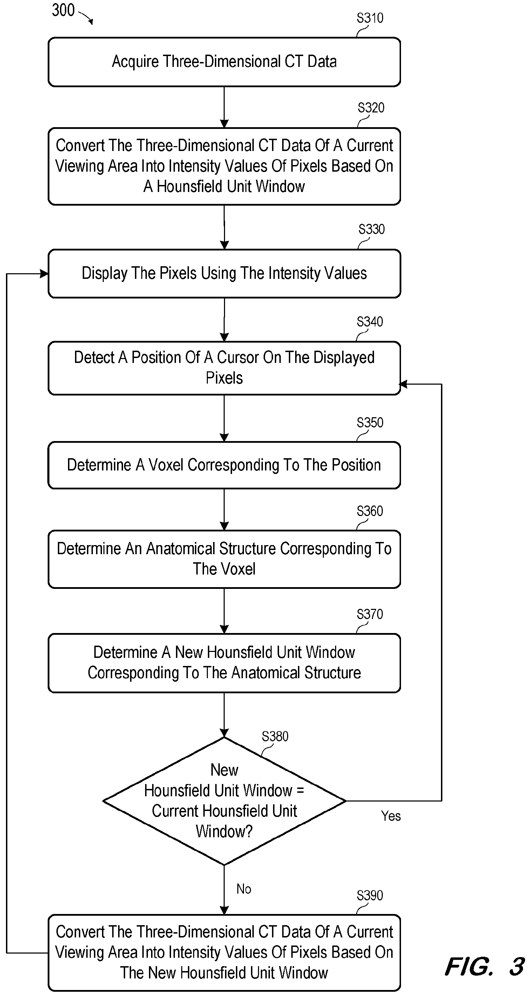

FIG. 3 is a flow diagram of process 300 to determine and apply different HU windows during viewing of a CT image according to some embodiments. Process 300 may be performed by any combination of hardware and software that is or becomes known. Program code embodying processes described herein may be stored by any one or more non-transitory tangible media, including a fixed disk, a volatile or non-volatile random-access memory, a DVD, a Flash drive, and a magnetic tape, and executed by any suitable processing unit, including but not limited to one or more microprocessors, microcontrollers, processor cores, and processor threads. Embodiments are not limited to the examples described below.

Initially, three-dimensional CT data is acquired at S310. The acquired CT data comprises voxels representing a volume such as a patient body. Each voxel is associated with an HU value which represents the radiodensity of material located at the voxel. The CT data may have been generated by a CT scanner at any time prior to S310. That is, a system executing process 300 need not be the same system which generates the CT data acquired at S310.

A current viewing area of the three-dimensional CT data is then determined at S320. The current viewing area is an area of the volume to be displayed to an operator. The current viewing area may be a slice of the volume taken through any desired angle of the volume. The current viewing area may be a slice located at a default position of the volume.

Also at S320, three-dimensional CT data of the current viewing area is converted into intensity values of pixels based on an HU window. According to some embodiments, a display consists of pixels which present a UI application. A portion of these pixels (e.g., a viewing window of the UI application) is intended to display an image based on the CT data.

For each pixel of this portion, S320 comprises determining the voxel of the current area to be represented by the pixel, determining an HU value of the voxel, and determining an intensity value of the pixel based on the HU value and an HU window (defined, e.g., as an HU window center and an HU window size). In some embodiments, a pixel may represent two or more adjacent voxels, in which case the intensity value of the pixel is determined based on the HU values of the two or more adjacent voxels and an HU window. The HU window used at S320 may be a default value and may be an operator-specific default value.

The pixels are displayed at S330 using their determined intensity values. As a result, the current viewing area of the volume is displayed in accordance with the HU window. According to some embodiments, an operator may operate the UI application to change the current viewing area of the volume (e.g., the current slice) to a new viewing area. Also, or alternatively, the operator may operate the UI application to move a cursor over the pixels displayed at S330.

A position of the cursor on the displayed pixels is detected at S340. The detected position may be represented by an identifier of a pixel which is co-located with the cursor. Next, a voxel corresponding to the detected position is determined at S350. Since the pixel intensity of each displayed pixel is determined based on an HU value of a voxel represented by the pixel, S350 may comprise determining the voxel whose HU value is represented by the pixel which is located at the detected cursor position.

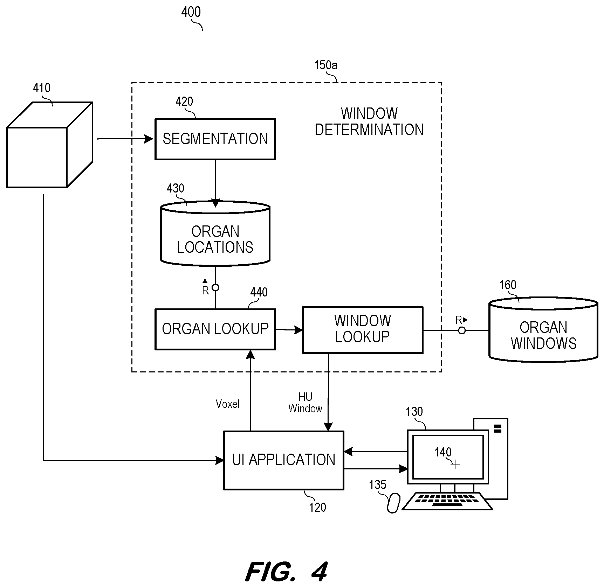

An anatomical structure corresponding to the voxel is determined at S360. FIG. 4 illustrates system 400 for determining an anatomical structure corresponding to a voxel according to some embodiments.

Window determination component 150a includes segmentation component 420. Segmentation component 420 receives CT data 410 and identifies anatomical structures therein and their locations as is known in the art. Generally, segmentation component 420 determines sub-volumes and corresponding anatomical structures located within the sub-volumes. Segmentation component 420 stores the locations of the sub-volumes and identifiers of their corresponding anatomical structures in organ locations 430.

Organ lookup component 440 receives an identifier of a voxel which was determined by UI application 120 at S350. Component 440 queries organ locations 430 for an anatomical structure at S360 based on the received voxel. In one example, organ locations 430 determines a sub-volume to which the voxel belongs and identifies an anatomical structure associated with the sub-volume.

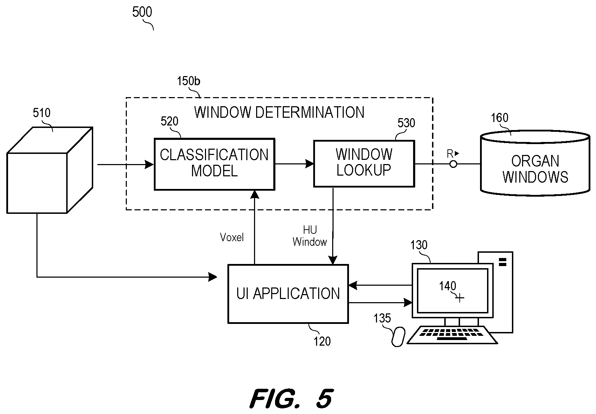

FIG. 5 illustrates system 500 for determining an anatomical structure based on a voxel at S360 according to some embodiments. In contrast to window determination component 150a of FIG. 4, window determination component 150b includes classification model 520. Classification model 520 may comprise any model trained to output an identifier of an anatomical structure based on input image data and a location of a selected voxel.

According to some embodiments, the input to classification model 520 is determined based on values of voxels of CT data which are proximate to and include the current voxel received from UI 120. According to some embodiments, the values comprise three orthogonal 27x27 grids of orthogonal planes at 4mm resolution and six 9x9x9 three-dimensional grids with resolutions of 2, 3, 5, 12, 28, 64 mm. A descriptor is created based on the values and input to model 520 at S360.

Model 520 may embody any neural network architecture providing a classification function. According to one example, model 520 includes residual networks with fully connected layers. Considering the descriptor as a flat array, the layers may comprise 1D linear layers with skip connections. Any activation function may be deployed between the linear layers, such as but not limited to the swish activation function.

Classification model 520 is trained using supervised learning as is known in the art. Generally, many images are acquired and anatomical structures within the images are determined using any suitable method(s). For each image, a descriptor is created for each voxel and the descriptor is associated with an anatomical structure corresponding to the voxel. The descriptors and corresponding anatomical structures are used to train and test model 520.

Classification model 520 outputs an identifier of an anatomical structure. Similarly, organ lookup component 440 of system 400 outputs an identifier of an anatomical structure. Returning to process 300, and for each of system 400 and 500, a new HU window is determined at S370 based on the identifier of the anatomical structure.

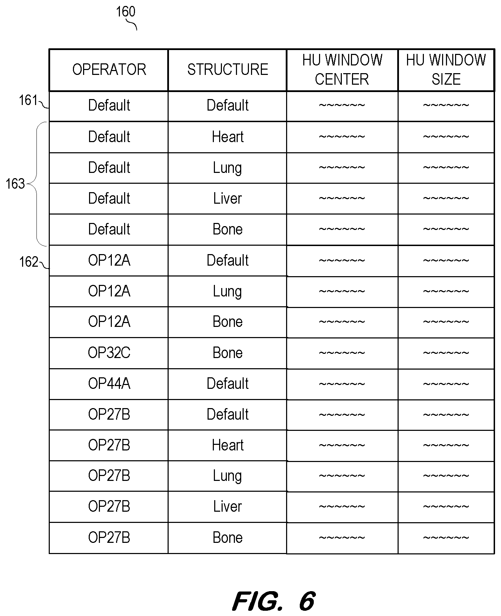

S370 may comprise performing a lookup on a data structure which associates anatomical structures with HU windows, such as organ windows data 160. FIG. 6 is a tabular representation of organ windows data 160 according to some embodiments. Each row of data 160 associates an operator identifier and an anatomical structure identifier with an HU window center and an HU window size.

Row 161 of data 160 associates a default operator and a default structure with HU window information. The HU window information of row 161 may comprise a default HU window which is used to convert HU values to pixel intensities in a case that the anatomical structure corresponding to the current cursor position is unknown (e.g., at S320) and data 160 does not include a row associating the current operator with a default anatomical structure. Row 162, in contrast, specifies an HU window which is used to convert HU values to pixel intensities in a case that the anatomical structure corresponding to the current cursor position is unknown and the current operator is associated with identifier OP12A.

Data 160 includes rows 163 specifying HU windows for different anatomical structures to be used in a case that data 160 does not include an operator-specific HU window for the anatomical structures. According to some embodiments, the HU window information of the non-operator-specific rows (i.e., 161 and 163) of data 160 may be pre-defined while the operator-specific rows may be generated and stored based on operator interactions with UI application 120.

Returning to process 300, it is determined at S380 whether the new HU window determined at S370 is identical (i.e., having the same center and size) to the HU window on which the currently-displayed pixel intensities are based. If so, flow returns to S340 to detect the cursor position as described above. If not, the CT data of the current viewing area is converted into intensity values of pixels based on the new HU window at S390. Similar to that described above with respect to S320, and for each pixel depicting CT data, the voxel represented by the pixel is determined, an HU value of the voxel is determined, and an intensity value of the pixel is determined based on the HU value and the new HU window.

Flow then returns to S330 to display the pixels using the newly-determined intensity values. The current viewing area of the volume is thereby displayed in accordance with the new HU window. Flow continues to thereafter cycle between S330 and S390 to determine the anatomical structure corresponding to the current cursor position, to determine whether to change the HU window underlying the currently-displayed pixel intensity values based on the anatomical structure, and, if it is determined to do so, to change the HU window and the currently-displayed pixel intensity values. Advantageously, by virtue of the features of system 400, system 500, or other suitable systems, the determination of the anatomical structure and changing of the HU window may occur in near real-time as an operator moves the cursor with respect to the displayed image.

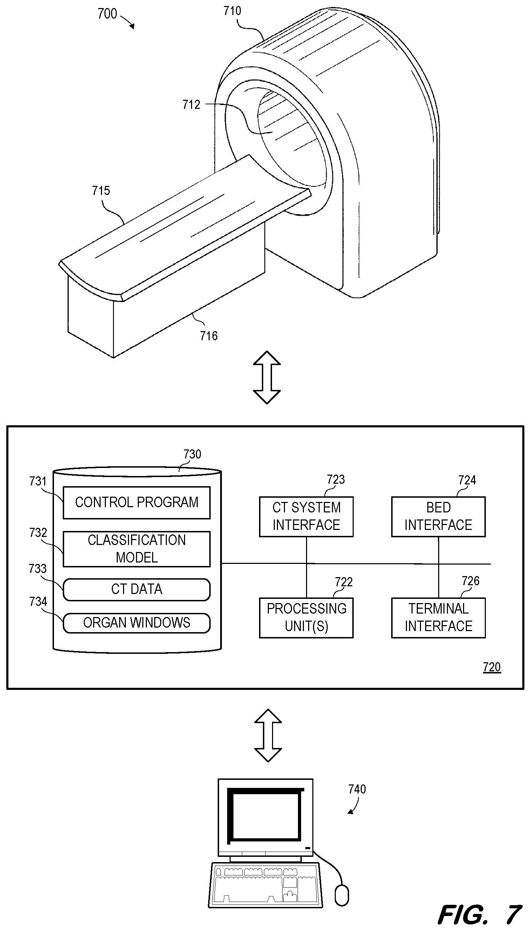

FIG. 7 illustrates CT scanner 700 to execute one or more of the processes described herein. Embodiments are not limited to scanner 700 and may be implemented, for example, by a multi-modality imaging system (e.g., PET/CT).

Scanner 700 includes gantry 710 defining bore 712. As is known in the art, gantry 710 houses CT imaging components for acquiring CT image data. The CT imaging components may include one or more x-ray tubes and one or more corresponding X-ray detectors.

Bed 715 and base 716 are operable to move a patient lying on bed 715 into and out of bore 712 before, during and after imaging. Movement of a patient into and out of bore 712 may allow scanning of the patient using the CT imaging elements. In some embodiments, bed 715 is configured to translate over base 716 and, in other embodiments, base 716 is movable along with or alternatively from bed 715. Bed 715 and base 716 may provide continuous bed motion and/or step- and-shoot motion during such scanning according to some embodiments.

Control system 720 may comprise any general-purpose or dedicated computing system. Accordingly, control system 720 includes one or more processing units 722 configured to execute program code and storage device 730 for storing the program code. Storage device 730 may comprise one or more fixed disks, solid-state random-access memory, and/or removable media (e.g., a thumb drive) mounted in a corresponding interface (e.g., a Universal Serial Bus port).

Storage device 730 stores program code of control program 731. One or more processing units 722 may execute control program 731 to control CT imaging elements of scanner 700 using CT system interface 723 and bed interface 724 to acquire CT data 733. Control program 731 may include program code executable to cause system 720 to perform process 300. For example, one or more processing units 722 may execute the program code to control terminal interface 726 to display an image on terminal 740 having pixel intensities determined based on CT data 733 and on an HU window specified in organ windows 734. Classification model 732 may be employed to determine an anatomical structure corresponding to a position of a cursor on terminal 740, and, if needed, the pixel intensities may be re-determined based on a new HU window specified in organ windows 734.

Each component of scanner 700 may include other elements which are necessary for the operation thereof, as well as additional elements for providing functions other than those described herein. Each functional component described herein may be implemented in computer hardware, in program code and/or in one or more computing systems executing such program code as is known in the art. Such a computing system may include one or more processing units which execute processor-executable program code stored in a memory system.

Those in the art will appreciate that various adaptations and modifications of the above-described embodiments can be configured without departing from the claims. Therefore, it is to be understood that the claims may be practiced other than as specifically described herein.

## Claims

1. An imaging system comprising:
an x-ray tube;
a detector for receiving photons emitted by the x-ray tube and generating CT data comprising Hounsfield Unit (HU) values for each of a plurality of voxels of a volume;
a display; and
a processing unit to:
instruct the display to present pixels having first pixel intensities determined based on the HU values of a first set of the plurality of voxels and a first HU window;
determine a position of a cursor on the display; and
in response to the determination of the position of the cursor, automatically:
determine an anatomical structure represented by a pixel corresponding to the position of the cursor;
determine a second HU window based on the anatomical structure, the second HU window being different from the first HU window;
determine second pixel intensities of the pixels based on the HU values of the first set of the plurality of voxels and the second HU window; and
instruct the display to present the pixels having the second pixel intensities.

2. The imaging system of Claim 1, the processing unit to:
determine a second position of the cursor on the display; and
in response to the determination of the second position of the cursor, automatically:
determine a second anatomical structure represented by a second pixel corresponding to the second position of the cursor;
determine a third HU window based on the second anatomical structure, the third HU window being different from the second HU window;
determine third pixel intensities of the pixels based on the HU values of the first set of the plurality of voxels and the third HU window; and
instruct the display to present the pixels having the third pixel intensities.

3. The imaging system of Claim 2, the processing unit to:
instruct the display to present pixels having fourth pixel intensities determined based on the HU values of a second set of the plurality of voxels and the third HU window;
determine a third position of the cursor on the display; and
in response to the determination of the third position of the cursor, automatically:
determine a third anatomical structure represented by a third pixel corresponding to the third position of the cursor;
determine a fourth HU window based on the third anatomical structure, the fourth HU window being different from the third HU window;
determine fifth pixel intensities of the pixels based on the HU values of the second set of the plurality of voxels and the fourth HU window; and
instruct the display to present the pixels having the fifth pixel intensities.

4. The imaging system of one of Claims 1 to 3, wherein determination of the anatomical structure represented by the pixel corresponding to the position of the cursor comprises:
determination of one of the first set of the plurality of voxels represented by the pixel; and
determination of the anatomical structure based on the voxel.

5. The imaging system of Claim 4, wherein determination of the anatomical structure based on the voxel comprises:
determination of a location of the voxel in the volume; and
determination of an anatomical structure located at the location of the voxel in the volume based on a segmentation map.

6. The imaging system of Claim 4, wherein determination of the anatomical structure based on the voxel comprises:
determination of HU values of a plurality of voxels proximate to the voxel in the volume;
determination of a descriptor based on the HU values;
input of the descriptor to a trained classification model; and
receipt of an identifier of an anatomical structure in response to the input of the descriptor.

7. The imaging system of one of Claims 1 to 6, the processing unit to:
instruct the display to present pixels having third pixel intensities determined based on the HU values of a second set of the plurality of voxels and the second HU window;
determine a second position of the cursor on the display; and
in response to the determination of the second position of the cursor, automatically:
determine a second anatomical structure represented by a second pixel corresponding to the second position of the cursor;
determine a third HU window based on the second anatomical structure, the third HU window being different from the second HU window;
determine third pixel intensities of the pixels based on the HU values of the second set of the plurality of voxels and the third HU window; and
instruct the display to present the pixels having the third pixel intensities.

8. A method comprising:
determining first pixel intensities based on Hounsfield Unit (HU) values of a first set of a plurality of voxels of a volume and a first HU window;
presenting pixels having the first pixel intensities on a display;
determining a position of a cursor on the display; and
in response to the determining the position of the cursor, automatically:
determining an anatomical structure corresponding to the position of the cursor;
determining a second HU window based on the anatomical structure, the second HU window being different from the first HU window;
determining second pixel intensities of the pixels based on the HU values of the first set of the plurality of voxels and the second HU window; and
changing the first pixel intensities of the displayed pixels to the second pixel intensities.

9. The method of Claim 8, further comprising:
determining a second position of the cursor on the display; and
in response to determining the second position of the cursor, automatically:
determining a second anatomical structure corresponding to the second position of the cursor;
determining a third HU window based on the second anatomical structure, the third HU window being different from the second HU window;
determining third pixel intensities of the pixels based on the HU values of the first set of the plurality of voxels and the third HU window; and
changing the second pixel intensities of the displayed pixels to the third pixel intensities.

10. The method of Claim 9, further comprising:
determining fourth pixel intensities based on the HU values of a second set of the plurality of voxels and the third HU window;
changing the third pixel intensities of the displayed pixels to the fourth pixel intensities;
determining a third position of the cursor on the display; and
in response to determining the third position of the cursor, automatically:
determining a third anatomical structure corresponding to the third position of the cursor;
determining a fourth HU window based on the third anatomical structure, the fourth HU window being different from the third HU window;
determining fifth pixel intensities of the pixels based on the HU values of the second set of the plurality of voxels and the fourth HU window; and
changing the fourth pixel intensities of the displayed pixels to the fifth pixel intensities.

11. The method of one of Claims 8 to 10, wherein determining the anatomical structure corresponding to the position of the cursor comprises:
determining one of the first set of the plurality of voxels corresponding to the position of the cursor; and
determining the anatomical structure based on the voxel.

12. The method of Claim 11, wherein determining the anatomical structure based on the voxel comprises:
determining a location of the voxel in the volume; and
determining an anatomical structure located at the location of the voxel in the volume based on a segmentation map.

13. The method of Claim 11, wherein determining the anatomical structure based on the voxel comprises:
determining HU values of a plurality of voxels proximate to the voxel in the volume;
determining a descriptor based on the HU values;
inputting the descriptor to a trained classification model; and
receiving an identifier of an anatomical structure in response to the input of the descriptor.

14. The method of one of Claims 8 to 13, further comprising:
determining third pixel intensities based on the HU values of a second set of the plurality of voxels and the second HU window;
changing the second pixel intensities of the displayed pixels to the third pixel intensities;
determining a second position of the cursor on the display; and
in response to determining the second position of the cursor, automatically:
determining a second anatomical structure corresponding to the second position of the cursor;
determining a third HU window based on the second anatomical structure, the third HU window being different from the second HU window;
determining fourth pixel intensities of the pixels based on the HU values of the second set of the plurality of voxels and the third HU window; and
changing the third pixel intensities of the displayed pixels to the fourth pixel intensities.

15. One or more non-transitory computer-readable media storing program code that, when executed by a computing system, causes the computing system to perform operations comprising:
determining first pixel intensities based on Hounsfield Unit (HU) values of a first set of a plurality of voxels of a volume and a first HU window;
presenting pixels having the first pixel intensities on a display;
determining a position of a cursor on the display;
determining an anatomical structure corresponding to the position of the cursor;
determining a second HU window based on the anatomical structure, the second HU window being different from the first HU window;
determining second pixel intensities of the pixels based on the HU values of the first set of the plurality of voxels and the second HU window; and
changing the first pixel intensities of the displayed pixels to the second pixel intensities.

16. The one or more non-transitory computer-readable media of Claim 15, the operations further comprising:
determining a second position of the cursor on the display;
determining a second anatomical structure corresponding to the second position of the cursor;
determining a third HU window based on the second anatomical structure, the third HU window being different from the second HU window;
determining third pixel intensities of the pixels based on the HU values of the first set of the plurality of voxels and the third HU window; and
changing the second pixel intensities of the displayed pixels to the third pixel intensities.

17. The one or more non-transitory computer-readable media of Claim 16, the operations further comprising:
determining fourth pixel intensities based on the HU values of a second set of the plurality of voxels and the third HU window;
changing the third pixel intensities of the displayed pixels to the fourth pixel intensities;
determining a third position of the cursor on the display;
determining a third anatomical structure corresponding to the third position of the cursor;
determining a fourth HU window based on the third anatomical structure, the fourth HU window being different from the third HU window;
determining fifth pixel intensities of the pixels based on the HU values of the second set of the plurality of voxels and the fourth HU window; and
changing the fourth pixel intensities of the displayed pixels to the fifth pixel intensities.

18. The one or more non-transitory computer-readable media of one of Claims 15 to 17, wherein determining the anatomical structure corresponding to the position of the cursor comprises:
determining one of the first set of the plurality of voxels corresponding to the position of the cursor; and
determining the anatomical structure based on the voxel.

19. The one or more non-transitory computer-readable media of Claim 18, wherein determining the anatomical structure based on the voxel comprises:
determining a location of the voxel in the volume; and
determining an anatomical structure located at the location of the voxel in the volume based on a segmentation map.

20. The one or more non-transitory computer-readable media of Claim 18, wherein determining the anatomical structure based on the voxel comprises:
determining HU values of a plurality of voxels proximate to the voxel in the volume;
determining a descriptor based on the HU values;
inputting the descriptor to a trained classification model; and
receiving an identifier of an anatomical structure in response to the input of the descriptor.
